# EUROPEAN PATENT APPLICATION

(11) **EP 4 693 329 A1**
(43) Date of publication of application: **11.02.2026**
(21) Application number: 25191220.0
(22) Date of filing: 23.07.2025
(51) Int. Cl.: G16H 30/40, G16H 40/67, A61C 9/00

(54) **INFORMATION PROCESSING SYSTEM, SERVER APPARATUS, AND CLIENT APPARATUS**

(30) Priority: 07.08.2024 JP 2024130674
(71) Applicant: J. MORITA MFG. CORP., Fushimi-ku Kyoto-shi, Kyoto 612-8533 (JP)
(72) Inventor: HAYASHI, Takeshi, Kyoto-shi, Kyoto 612-8533 (JP); SANO, Masayuki, Kyoto-shi, Kyoto 612-8533 (JP); WAKAZOME, Naonori, Kyoto-shi, Kyoto 612-8533 (JP); OKADA, Takuya, Kyoto-shi, Kyoto 612-8533 (JP)
(74) Representative: Müller Hoffmann & Partner

(57) **Abstract**

An information processing system, a server apparatus, and a client apparatus that allow reduction in introduction cost, provide high convenience, and process three-dimensional data are provided. The three-dimensional scanner system includes a scanner, a server apparatus, and a client apparatus. The scanner scans an object to sequentially acquire pieces of three-dimensional data of the object. When joining the pieces of three-dimensional data based on an overlapping portion of pieces of three-dimensional data sequentially transmitted from the scanner and generating combined data indicating a three-dimensional shape of the object, the server apparatus generates, as difference data, a difference between pieces of combined data obtained before and after joining pieces of three-dimensional data together. The client apparatus accumulates pieces of difference data sequentially transmitted from the server apparatus, and generates a two-dimensional image of the object as viewed from any point of view based on the accumulated pieces of difference data.

## Description

### CROSS REFERENCE TO RELATED APPLICATIONS

This application is based on Japanese Patent Application No. 2024-130674 filed on August 7, 2024 with the Japan Patent Office.

### BACKGROUND

### Field

The present disclosure relates to an information processing system, a server apparatus, and a client apparatus.

### Description of the Background Art

In the dental field, a scanner for acquiring a three-dimensional shape of teeth has been developed in order to digitally design a prosthesis and the like on a computer (for example, Japanese Patent No. 5654583). The scanner, for example, acquires a three-dimensional shape of a surface of an object by using the technique of a focusing method, generates three-dimensional data, calculates a two-dimensional projection view based on the three-dimensional data, and causes a display to show the two-dimensional projection view. Thus, a computing apparatus connected to the scanner needs to perform high-load processing.

### SUMMARY

When a plurality of scanners are introduced in a clinic, however, it is necessary to prepare the same number of computing apparatuses for performing high-load processing as the number of scanners, which leads to a problem of an increase in introduction cost. Further, when one scanner is used at a plurality of places (for each chair unit) in a clinic, it is necessary to move not only the scanner but also the computing apparatus in one set to the place where the scanner is to be used, so that convenience may decrease.

The present disclosure has been made to solve the above-described problems, and an object thereof is to provide an information processing system, a server apparatus, and a client apparatus that allow reduction in an introduction cost, provide high convenience, and process three-dimensional data.

An information processing system according to the present disclosure is an information processing system configured to process three-dimensional data. The information processing system includes a scanner, a server apparatus, and a client apparatus. The scanner is configured to scan an object to sequentially acquire pieces of three-dimensional data of the object. The server apparatus is configured to, when joining pieces of three-dimensional data based on an overlapping portion of the pieces of three-dimensional data sequentially transmitted from the scanner and generating combined data indicating a three-dimensional shape of the object, generate, as difference data, a difference between pieces of the combined data obtained before and after joining the pieces of three-dimensional data together. The client apparatus is configured to accumulate pieces of the difference data sequentially transmitted from the server apparatus, and generate a two-dimensional image of the object as viewed from any point of view based on the accumulated pieces of the difference data.

A server apparatus according to the present disclosure is a server apparatus configured to transmit and receive data to and from a client apparatus included in an information processing system configured to process three-dimensional data. The server apparatus includes a reception unit, a computing unit, and a transmission unit. The reception unit is configured to receive pieces of three-dimensional data sequentially transmitted from a scanner configured to scan an object to sequentially acquire pieces of three-dimensional data of the object. The computing unit is configured to, when joining pieces of three-dimensional data together based on an overlapping portion of the pieces of three-dimensional data sequentially received by the reception unit and generating combined data indicating a three-dimensional shape of the object, generate, as difference data, a difference between pieces of the combined data obtained before and after joining the pieces of three-dimensional data together. The transmission unit is configured to accumulate pieces of the difference data generated by the computing unit, and transmit the accumulated pieces of the difference data to a client apparatus configured to generate a two-dimensional image of the object as viewed from any point of view based on the accumulated pieces of the difference data.

A client apparatus according to the present disclosure is a client apparatus configured to transmit and receive data to and from a server apparatus included in an information processing system configured to process three-dimensional data. The client apparatus includes a reception unit, a computing unit, and a display unit. The reception unit is configured to, when joining pieces of three-dimensional data together based on an overlapping portion of pieces of three-dimensional data sequentially transmitted from a scanner configured to scan an object to sequentially acquire pieces of three-dimensional data of the object, and generating combined data indicating a three-dimensional shape of the object, receive difference data transmitted from the server apparatus configured to generate, as the difference data, a difference between pieces of the combined data obtained before and after joining the pieces of three-dimensional data together. The computing unit is configured to accumulate pieces of the difference data received by the reception unit and generate a two-dimensional image of the object as viewed from any point of view based on the accumulated pieces of the difference data. The display unit is configured to display the two-dimensional image.

The foregoing and other objects, features, aspects, and advantages of the present disclosure will become apparent from the following detailed description of the present disclosure when taken in conjunction with the accompanying drawings.

### BRIEF DESCRIPTION OF THE DRAWINGS

Fig. 1 is a schematic diagram showing a configuration of a three-dimensional scanner system according to an embodiment.
Fig. 2 is a block diagram showing a configuration of a server apparatus according to the embodiment.
Fig. 3 is a block diagram showing a configuration of a client apparatus according to the embodiment.
Fig. 4 is a sequence diagram for illustrating processing of three-dimensional data in the three-dimensional scanner system according to the embodiment.
Fig. 5 is a schematic diagram for illustrating an example of processing of the three-dimensional data in the three-dimensional scanner system according to the embodiment.
Fig. 6 is a sequence diagram for illustrating a deletion process in the three-dimensional scanner system according to the embodiment.
Fig. 7 is a schematic diagram for illustrating an example of the deletion process in the three-dimensional scanner system according to the embodiment.
Fig. 8 is a sequence diagram for illustrating an unnecessary object deletion process in the three-dimensional scanner system according to the embodiment.
Fig. 9 is a schematic diagram for illustrating an example of the unnecessary object deletion process in the three-dimensional scanner system according to the embodiment.
Fig. 10 is a sequence diagram for illustrating a positional deviation correction process in the three-dimensional scanner system according to the embodiment.
Fig. 11 is a schematic diagram for illustrating an example of the positional deviation correction process in the three-dimensional scanner system according to the embodiment.
Fig. 12 is a sequence diagram for illustrating a margin line process in the three-dimensional scanner system according to the embodiment.
Fig. 13 is a sequence diagram for illustrating an example of a dental arch scanning process in the three-dimensional scanner system according to the embodiment.
Fig. 14 is a schematic diagram showing a configuration of a three-dimensional scanner system according to a modification.

### DESCRIPTION OF THE PREFERRED EMBODIMENTS

An information processing system according to an embodiment will be hereinafter described with reference to the accompanying drawings. In the embodiment, a three-dimensional scanner system used in a dental medical care will be described as one exemplary embodiment of the information processing system. The three-dimensional scanner system is an intra-oral scanner system for acquiring a three-dimensional shape of teeth inside an oral cavity. The information processing system according to the embodiment is not limited to the intra-oral scanner system but is applicable also to other information processing systems having the same configuration as described above for processing three-dimensional data, and applicable also to an information processing system, for example, that images the inside of a human ear other than the inside of the oral cavity and processes three-dimensional data about the inside of an outer ear.

The field in which the information processing system according to the embodiment is used is not limited to dentistry but is applicable in every medical field including ophthalmology, otolaryngology, radiology, and veterinary science, and the like, and applicable also in other fields such as industrial and engineering fields other than the medical fields. In the present disclosure, the term "medical care" also includes meanings of diagnosis and treatment.

### [Configuration of Three-Dimensional Scanner System]

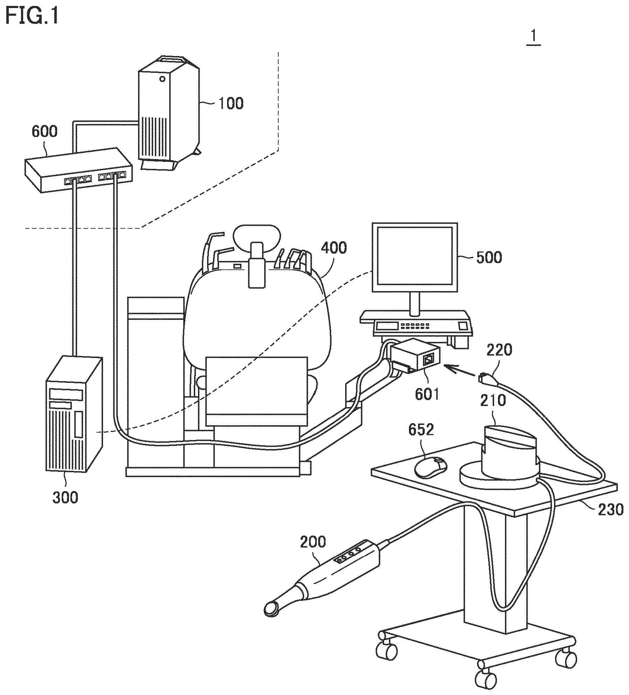

Fig. 1 is a schematic diagram showing a configuration of a three-dimensional scanner system 1 according to an embodiment. As shown in Fig. 1, three-dimensional scanner system 1 includes a server apparatus 100, a scanner 200, and a client apparatus 300. Server apparatus 100 serves as a computing apparatus for executing high-load processing, and performs a process of joining pieces of three-dimensional data, a process of generating mesh data of a three-dimensional shape, and the like. Server apparatus 100 is installed in a place in a clinic (for example, a spare room or a storeroom in a clinic) different from a place where medical care is provided, or installed in a place outside the clinic. Server apparatus 100 may be a cloud server.

Scanner 200 has a probe portion that is inserted into an oral cavity to project light having a pattern onto an object such as a tooth, and measure light reflected from the object onto which the pattern is projected, to thereby acquire data of a three-dimensional shape of the object based on the principle of a focusing method. Note that the configuration of scanner 200 is not limited to a configuration for acquiring data of the three-dimensional shape of the object based on the principle of the focusing method, but scanner 200 may have a configuration adopting other principles as long as data of the three-dimensional shape of the object is acquired, for example, based on an optical method such as the principle of the confocal method or the like.

Scanner 200 is connected to a cradle 210 through a wired cable, and transmits the data of the three-dimensional shape of the object acquired through the wired cable to cradle 210. Cradle 210 is network-connected to server apparatus 100 through a LAN cable 220. Specifically, as shown in Fig. 1, cradle 210 is network-connected to server apparatus 100 via a network hub 600 by insertion of LAN cable 220 into an insertion port 601 provided in each chair unit 400.

Network hub 600 preferably has, for example, a Power over Ethernet (PoE) function. If network hub 600 has the PoE function, electric power can be supplied to scanner 200 and cradle 210 through LAN cable 220. When electric power can be supplied to scanner 200 and cradle 210 through LAN cable 220, supply of electric power to cradle 210 through an AC adapter or the like is not required.

Supply of electric power from cradle 210 to scanner 200 is implemented through a wired cable connecting cradle 210 and scanner 200. However, in a configuration in which scanner 200 and cradle 210 are wirelessly connected to each other and the data of the three-dimensional shape of the object acquired by scanner 200 is transmitted to cradle 210, scanner 200 needs to have a storage battery incorporated therein. The storage battery incorporated in scanner 200 may be charged when scanner 200 is placed on cradle 210.

Client apparatus 300 causes a display device 500 to display the data of the three-dimensional shape processed by server apparatus 100. Client apparatus 300 performs a process under a load lower than that in the case of server apparatus 100, such as a process of causing display device 500 to display three-dimensional data. Thus, the performance specifications required for client apparatus 300 are lower than that required for server apparatus 100, and the cost thereof is also low. For example, one server apparatus 100 is installed in a clinic, whereas client apparatus 300 is installed in each chair unit 400.

As described above, by dividing three-dimensional scanner system 1 into server apparatus 100 and client apparatus 300, processes can be distributed therebetween such that high-load processes and low-load processes can be allocated to server apparatus 100 and client apparatus 300, respectively. In general, since a computing apparatus with higher performance specifications is larger in size and heavier in weight, it is difficult to relocate such an apparatus in a clinic. On the other hand, since a computing apparatus with lower performance specifications is smaller in size and lighter in weight, it is easy to relocate such an apparatus in a clinic. Thus, server apparatus 100 is installed in a space such as a spare room or a storeroom in a clinic that are different from a medical examination and treatment room, and client apparatus 300 is disposed in each chair unit 400 to cause display device 500 to show the three-dimensional shape of an object that is being scanned. In this way, three-dimensional scanner system 1 allows a reduction in introduction cost and can provide a highly convenient information processing system.

When LAN cable 220 is inserted into insertion port 601 provided in each chair unit 400, scanner 200 can scan an object while moving between chair units 400. Specifically, scanner 200 and cradle 210 are placed on a wagon 230 shown in Fig. 1, wagon 230 is moved to chair unit 400 on which scanner 200 is used, and LAN cable 220 is inserted into insertion port 601 of this chair unit 400. An input device such as a mouse 652 may be placed on wagon 230 and used to optionally change the point of view of the image of the object displayed on display device 500.

Three-dimensional scanner system 1 has a configuration divided into server apparatus 100 that processes the data of the three-dimensional shape and client apparatus 300 that causes display device 500 to show an image of the data of the three-dimensional shape (a divided configuration). Instead of providing two computing apparatuses such as server apparatus 100 and client apparatus 300, however, three-dimensional scanner system 1 may have a configuration in which one computing apparatus has a function of server apparatus 100 and a function of client apparatus 300 (a stand-alone configuration). When three-dimensional scanner system 1 is configured in a stand-alone configuration, it is not necessary for dealers to prepare a lineup of several computing apparatuses, so that labor and cost for system maintenance and management can be reduced. Further, when three-dimensional scanner system 1 is configured in a stand-alone configuration, an object can be scanned also at a destination visited for a home-visit medical care simply by carrying scanner 200 and a computing apparatus configured to have the functions of server apparatus 100 and client apparatus 300. On the other hand, even when three-dimensional scanner system 1 is configured in a divided configuration, but if three-dimensional scanner system 1 can be connected through the Internet connection to server apparatus 100 installed in a clinic, a cloud, or the like, an object can be scanned also at a visited destination simply by carrying client apparatus 300 and scanner 200 to the destination.

Further, three-dimensional scanner system 1 is not limited to a configuration in which an application program for processing the data of the three-dimensional shape is introduced into server apparatus 100, and an application program for causing display device 500 to display an image of the data of the three-dimensional shape is introduced into client apparatus 300. For example, three-dimensional scanner system 1 may have a configuration in which server apparatus 100 functions as a Web server, and a Web application program running on a Web browser is introduced into client apparatus 300. Introducing a Web application program instead of a dedicated application program into client apparatus 300 eliminates the need to install a dedicated application program and makes it easy to upgrade the program. Further, since the Web application program runs on a browser, it can be used in computing apparatuses different in configuration (for example, a desktop personal computer (PC), a notebook PC, a tablet terminal, a smartphone, and the like), and also, the operating system (OS) (for example, Windows (registered trademark), Mac OS (registered trademark), Linux (registered trademark), Android (registered trademark), Chrome OS (registered trademark), and the like) that runs thereon is also not limited (multi-platform compatible).

Three-dimensional scanner system 1 does not adopt a remote desktop function allowing for transmission and reception, through the Internet connection, of the information that is input on a desktop screen at an access destination or input via a keyboard, a mouse or the like at an access source. In three-dimensional scanner system 1, server apparatus 100 generates difference data that is a difference between pieces of combined data obtained before and after joining pieces of three-dimensional data together, and then, transmits the difference data to client apparatus 300, as will be described later. Thus, three-dimensional scanner system 1 is less likely to have problems such as an inability to display an object image on display device 500 and a delay in display due to a performance decrease under the influence of the line speed on the Internet connection. Note that the difference data is point cloud data or mesh data composed of points each indicating a three-dimensional coordinate position.

Further, in three-dimensional scanner system 1, when client apparatus 300 has a failure and is replaced with another computing apparatus, only the Web browser needs to be operated but a dedicated application program does not need to be reinstalled. Client apparatus 300 only needs to have performance specifications allowing only a Web browser to operate and does not require a computing apparatus having high performance specifications, with the result that the introduction cost can be suppressed.

Further, even if the network is disconnected during scanning to prevent client apparatus 300 from communicating with server apparatus 100, three-dimensional scanner system 1 can cause client apparatus 300 to perform a drawing process, for example, for rotating, scaling up and down, and moving the three-dimensional shape of the object based on the pieces of difference data accumulated in client apparatus 300, as will be described later. Further, a Web application program is often used for an application program for giving an explanation to an image management system or a patient, and an application program for transmitting and receiving data (three-dimensional data of an object, a dental engineering instruction manual, or the like), a message, or the like to and from a dental clinic, a dental laboratory or the like. Further, the Web application programs used for switching tabs of the Web browser can be easily switched since the Web application programs are often on the same network in a clinic. Thus, the Web application program is adopted as an application program to be run on client apparatus 300, so that compatibility is further enhanced.

If chair unit 400 has a computing apparatus installed therein, this computing apparatus can be used as client apparatus 300. Further, a display device provided in chair unit 400 can be used as display device 500. By causing the display device provided in chair unit 400 to display the image of the object that is being scanned, the movement of the operator's (user's) line of sight can be reduced. Further, scanner 200 does not need to be placed on wagon 230 but may be placed on a tray of chair unit 400. In this case, wagon 230 is not required, and thus, occupying much space in the medical examination and treatment room is prevented. Further, in the case where a Web application program is adopted as an application program to be run on client apparatus 300, a license dongle should only be connected solely to server apparatus 100, and each client apparatus 300 does not need to have a license dongle. In other words, when scanner 200 is moved to another chair unit 400, the license dongle does not need to be moved from the currently connected client apparatus 300 to another client apparatus 300 at its destination for connection thereto.

### [Configuration of Server Apparatus]

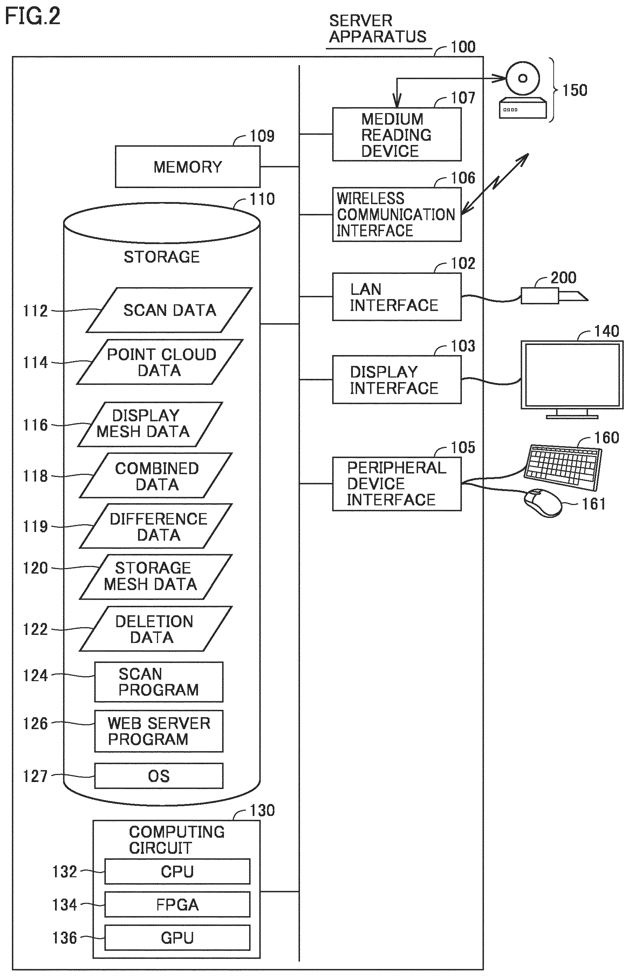

An example of a hardware configuration of server apparatus 100 according to the present embodiment will be hereinafter described with reference to Fig. 2. Fig. 2 is a block diagram showing a configuration of server apparatus 100 according to the embodiment. Server apparatus 100 may be implemented, for example, by a general-purpose computer or by a computer dedicated to three-dimensional scanner system 1.

As shown in Fig. 2, server apparatus 100 includes, as main hardware elements, a LAN interface 102, a display interface 103, a peripheral device interface 105, a wireless communication interface 106, a medium reading device 107, a memory 109, a storage 110, and a computing circuit 130.

LAN interface 102 is network-connected via network hub 600 shown in Fig. 1 to scanner 200, client apparatus 300, other computing apparatuses arranged locally in a clinic, the Internet, and the like. Fig. 2 shows scanner 200 to be directly connected to LAN interface 102. LAN interface 102 serves as an interface for connecting scanner 200, and implements input and output of data between server apparatus 100 and client apparatus 300, and also between server apparatus 100 and another locally arranged computing apparatus, cloud server, and the like.

Display interface 103 serves as an interface for connecting a display device 140 for server apparatus 100, and implements input and output of data between server apparatus 100 and display device 140. Display device 140 is configured, for example, of a liquid crystal display (LCD) or an organic electroluminescence (EL) display.

Peripheral device interface 105 serves as an interface for connecting peripheral devices such as a keyboard 160 and a mouse 161 for server apparatus 100, and implements input and output of data between server apparatus 100 and the peripheral devices.

Through wireless communication, wireless communication interface 106 transmits and receives data to and from devices disposed in a clinic, other locally arranged computing apparatuses, cloud servers, and the like. Wireless communication interface 106 is compatible with any communication scheme such as mobile communications (4G, 5G, and the like), wireless local area network (LAN), Bluetooth (registered trademark), and the like.

Medium reading device 107 reads various pieces of data and various programs stored in a removable disk 150.

Memory 109 provides a storage area in which a program code, a work memory, and the like are temporarily stored when computing circuit 130 executes any program. Memory 109 is configured, for example, of a volatile memory device such as a dynamic random access memory (DRAM) or a static random access memory (SRAM).

Storage 110 provides a storage area in which various pieces of data such as three-dimensional data of an object and various programs are stored. Storage 110 is configured, for example, of a nonvolatile memory device such as a hard disk or a solid state drive (SSD).

Storage 110 stores scan data 112, point cloud data 114, display mesh data 116, combined data 118, difference data 119, storage mesh data 120, deletion data 122, a scan program 124, a Web server program 126, and an operating system (OS) 127.

Scan data 112 is three-dimensional data acquired by scanner 200. Point cloud data 114 is data obtained by extracting point cloud information about an object from scan data 112. Display mesh data 116 is mesh data generated from scan data 112 obtained at the start of scanning, and is also data to be combined with pieces of scan data 112 that are subsequently transmitted sequentially from scanner 200. Combined data 118 is data obtained by combining display mesh data 116 with pieces of scan data 112 that are sequentially transmitted from scanner 200.

Difference data 119 is a difference between pieces of the combined data obtained before and after joining pieces of scan data 112 together. Storage mesh data 120 is mesh data reconstructed with all pieces of scan data 112 acquired by scanner 200 after the end of scanning. Deletion data 122 is data that is set in advance in order to specify an unnecessary object (for example, a finger, a treatment instrument, or the like) that is captured during scanning of an object and included in a resultant image. Scan program 124 is a program for processing three-dimensional data in server apparatus 100 during and after scanning of an object. Web server program 126 is a program for causing server apparatus 100 to function as a Web server.

Computing circuit 130, which is an example of a computer, is a computing entity that executes various programs to execute various processes in server apparatus 100. Computing circuit 130 is configured, for example, of a central processing unit (CPU) 132, a field-programmable gate array (FPGA) 134, a graphics processing unit (GPU) 136, and the like.

### [Configuration of Client Apparatus]

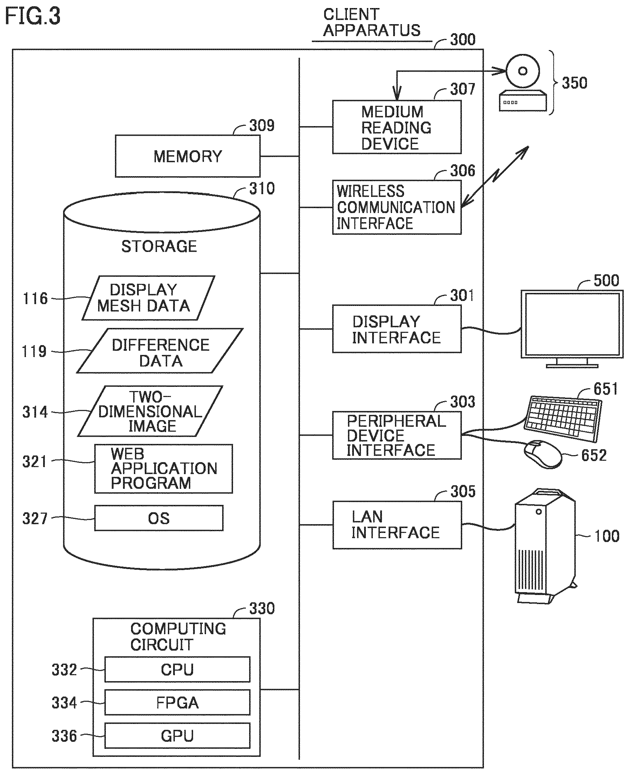

An example of a hardware configuration of client apparatus 300 according to the present embodiment will be hereinafter described with reference to Fig. 3. Fig. 3 is a block diagram showing a configuration of client apparatus 300 according to the embodiment. Client apparatus 300 may be implemented, for example, by a general-purpose computer or by a dedicated computer connected to chair unit 400.

As shown in Fig. 3, client apparatus 300 includes, as main hardware elements, a display interface 301, a peripheral device interface 303, a LAN interface 305, a wireless communication interface 306, a medium reading device 307, a memory 309, a storage 310, and a computing circuit 330.

Display interface 301 serves as an interface for connecting display device 500, and implements input and output of data between client apparatus 300 and display device 500. Display device 500 is configured, for example, of an LCD, an organic EL display, or the like.

Peripheral device interface 303 serves as an interface for connecting peripheral devices such as a keyboard 651 and mouse 652, and implements input and output of data between client apparatus 300 and each peripheral device.

LAN interface 305 is network-connected via network hub 600 shown in Fig. 1 to server apparatus 100, other computing apparatuses locally arranged in a clinic, the Internet, and the like. Fig. 3 shows server apparatus 100 to be directly connected to LAN interface 305. LAN interface 305 implements input and output of data between client apparatus 300 and server apparatus 100, and also between client apparatus 300 and another locally arranged computing apparatus, cloud server, and the like.

Through wireless communication, wireless communication interface 306 transmits and receives data to and from devices arranged in a clinic, other locally arranged computing apparatuses, cloud servers, and the like. Wireless communication interface 306 is compatible with any communication scheme such as mobile communications (4G, 5G, and the like), wireless LAN, Bluetooth (registered trademark), and the like.

Medium reading device 307 reads various pieces of data such as scan information stored in removable disk 350.

Memory 309 provides a storage area in which a program code, a work memory, and the like are temporarily stored when computing circuit 330 executes any program. Memory 309 is configured, for example, of a volatile memory device such as a DRAM or an SRAM.

Storage 310 provides a storage area in which various pieces of data and various programs are stored that are necessary for a process of causing display device 500 to display an image of an object obtained during and after scanning. Storage 310 is configured, for example, of a nonvolatile memory device such as a hard disk or an SSD.

Storage 310 stores display mesh data 116, difference data 119, a two-dimensional image 314, a Web application program 321, and an OS 327.

Display mesh data 116 is mesh data generated from scan data 112 obtained at the start of scanning, and is also data transmitted from server apparatus 100 after the start of scanning. Difference data 119 is a difference between pieces of the combined data obtained before and after joining pieces of scan data 112 together, and is also pieces of data sequentially transmitted from server apparatus 100 during scanning. Two-dimensional image 314 is an image of an object as viewed from any point of view based on the accumulated pieces of difference data 119. Web application program 321 is an application program that runs on a Web browser and causes display device 500 to display two-dimensional image 314 of an object.

Computing circuit 330, which is an example of a computer, is a computing entity that executes various programs to execute various processes such as a process of causing display device 500 to display two-dimensional image 314 of an object based on the data of the three-dimensional shape processed by server apparatus 100. Computing circuit 330 is configured, for example, of a CPU 332, an FPGA 334, a GPU 336, and the like.

### [Process in Three-Dimensional Scanner System]

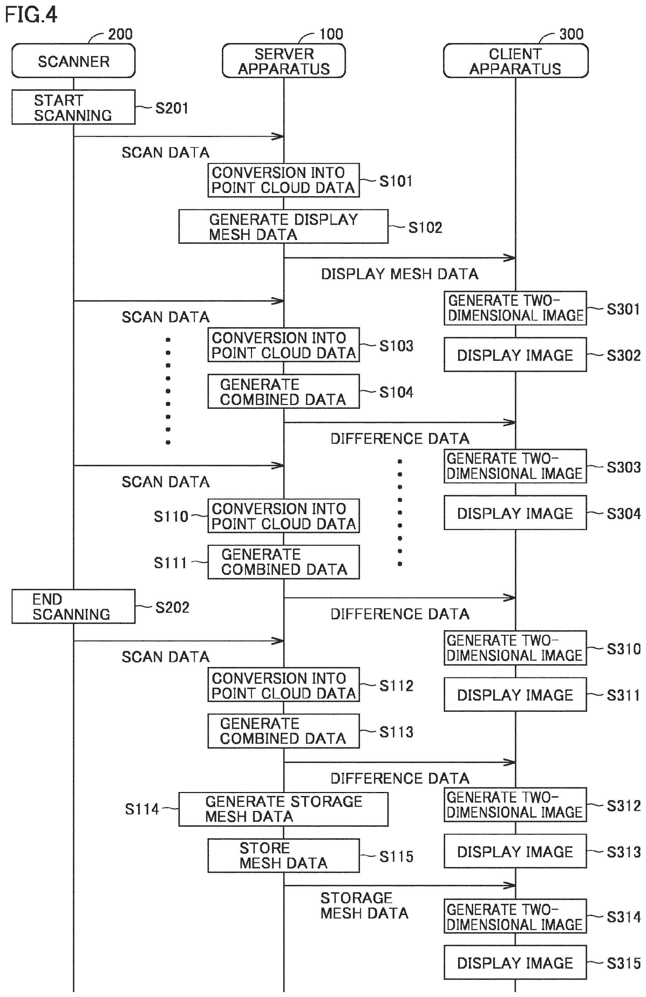

The following describes a process in three-dimensional scanner system 1 with reference to a sequence diagram. Fig. 4 is a sequence diagram for illustrating processing of three-dimensional data in three-dimensional scanner system 1 according to the embodiment. First, upon acceptance of an operation to start scanning by an operator, scanner 200 starts to acquire scan data 112 (three-dimensional data) of an object inside an oral cavity (S201). The object is, for example, a dental arch inside an oral cavity or a model of the dental arch.

Scanner 200 scans the dental arch inside the oral cavity to sequentially acquire pieces of scan data 112 of the dental arch, and sequentially transmits the acquired pieces of scan data 112 to server apparatus 100. Server apparatus 100 converts the pieces of scan data 112 sequentially transmitted from scanner 200 into point cloud data 114 (conversion into point cloud data: S101). Note that point cloud data 114 includes: position information (coordinates of each of axes in the vertical direction, the horizontal direction, and the height direction) of each of points indicating the surface shape of the dental arch; and color data indicating the color of each point. In a process of converting scan data 112 into point cloud data 114, server apparatus 100 may also perform a process of removing noise points from point cloud data 114 and a process of smoothing point cloud data 114. Further, server apparatus 100 may perform an image recognition process on each portion of the acquired point cloud data 114 to recognize which portion of point cloud data 114 corresponds to which part of the dental arch.

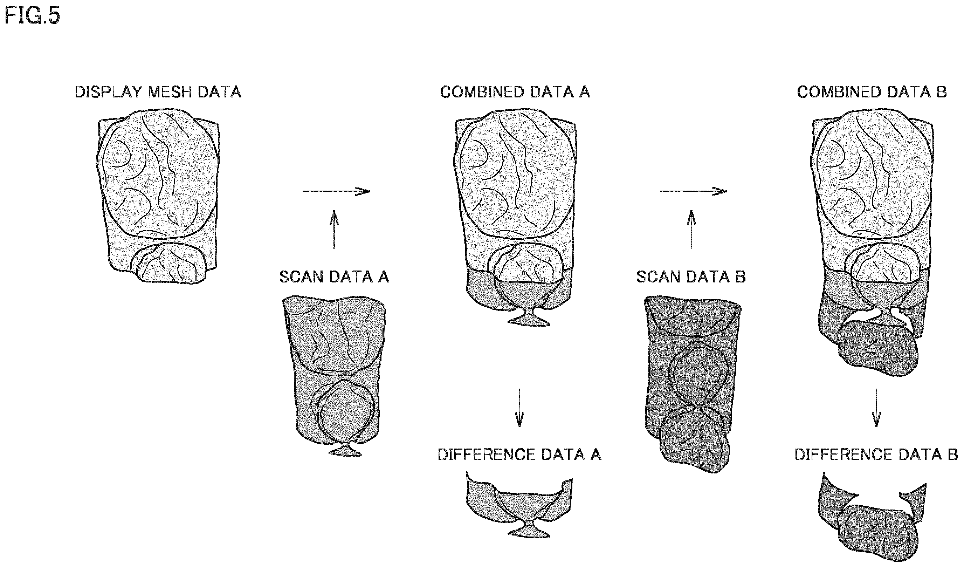

Server apparatus 100 generates display mesh data 116 corresponding to point cloud data 114 (S102). Display mesh data 116 is the first combined data obtained by sequentially joining pieces of scan data together. Specifically, server apparatus 100 generates display mesh data 116 from scan data 112 obtained at the start of scanning, as shown in Fig. 5. Fig. 5 is a schematic diagram for illustrating an example of processing of three-dimensional data in three-dimensional scanner system 1 according to the embodiment. When scanner 200 scans the dental arch, a plurality of pieces of scan data 112 are acquired at substantially the same position. Display mesh data 116 is generated using some of the pieces of acquired scan data 112 (for example, one piece of the first acquired scan data 112) instead of using all of the pieces of acquired scan data 112. Therefore, server apparatus 100 that generates display mesh data 116 operates under a low load, so that display mesh data 116 can be generated in real time.

Server apparatus 100 transmits the generated display mesh data 116 to client apparatus 300. Based on the received display mesh data 116, client apparatus 300 generates two-dimensional image 314 of the scanned dental arch as viewed from any point of view (S301). Client apparatus 300 generates two-dimensional image 314 of the dental arch scanned, for example, from a point of view input by the operator using mouse 652. Client apparatus 300 causes display device 500 to display the generated two-dimensional image 314 (S302). Specifically, client apparatus 300 calculates a two-dimensional projection view of display mesh data 116 as viewed from any point of view, and causes display device 500 to display the obtained two-dimensional projection view as two-dimensional image 314. For example, an algorithm such as perspective projection or parallel projection can be used to calculate the two-dimensional projection view.

Then, server apparatus 100 converts scan data 112 newly transmitted from scanner 200 into point cloud data 114 (conversion into point cloud data: S103). Specifically, server apparatus 100 receives scan data A as shown in Fig. 5 and converts scan data A into point cloud data 114. Server apparatus 100 joins two pieces of data based on an overlapping portion between display mesh data 116 and newly acquired scan data 112 to generate combined data 118 (S104). Specifically, as shown in Fig. 5, server apparatus 100 generates combined data A obtained by joining scan data A to the display mesh data based on the overlapping portion between the display mesh data and scan data A. Note that combined data 118 is generated using some of the pieces of acquired scan data 112 (for example, one piece of the first acquired scan data 112) instead of using all of the pieces of acquired scan data 112. Therefore, server apparatus 100 that generates combined data 118 operates under a low load, so that combined data 118 can be generated in real time.

When generating combined data 118, server apparatus 100 generates, as difference data 119, a difference between pieces of combined data 118 obtained before and after joining pieces of scan data 112 together, and transmits difference data 119 to client apparatus 300. Specifically, server apparatus 100 generates a newly joined portion of combined data A as difference data A as shown in Fig. 5, and transmits difference data A to client apparatus 300.

Client apparatus 300 accumulates pieces of difference data 119 transmitted from server apparatus 100, and generates two-dimensional image 314 of the dental arch as viewed from any point of view based on the accumulated pieces of difference data 119 (S303). Specifically, client apparatus 300 produces combined data A obtained by joining difference data A to the display mesh data as shown in Fig. 5, and generates two-dimensional image 314 of the dental arch scanned from the point of view input by the operator with mouse 652. Client apparatus 300 causes display device 500 to display the generated two-dimensional image 314 (S304). Specifically, client apparatus 300 calculates a two-dimensional projection view of combined data 118 as viewed from any point of view, and causes display device 500 to display the obtained two-dimensional projection view as two-dimensional image 314. For example, an algorithm such as perspective projection or parallel projection can be used to calculate the two-dimensional projection view. Since client apparatus 300 receives only differential data A instead of combined data A from server apparatus 100, the amount of data transmitted from server apparatus 100 can be reduced.

Further, server apparatus 100 converts scan data 112 newly transmitted from scanner 200 into point cloud data 114 (conversion into point cloud data: S110). Specifically, server apparatus 100 receives scan data B as shown in Fig. 5, and converts scan data B into point cloud data 114. Server apparatus 100 joins two pieces of data based on the overlapping portion between combined data 118 and the newly acquired scan data 112 to generate new combined data 118 (S111). Specifically, server apparatus 100 generates combined data B obtained by joining scan data B to combined data A based on the overlapping portion between combined data A and scan data B as shown in Fig. 5.

When generating combined data 118, server apparatus 100 generates, as difference data 119, a difference between pieces of combined data 118 obtained before and after joining pieces of scan data 112 together, and transmits difference data 119 to client apparatus 300. Specifically, server apparatus 100 generates a newly joined portion of combined data B as difference data B as shown in Fig. 5, and transmits difference data B to client apparatus 300.

Client apparatus 300 accumulates pieces of difference data 119 transmitted from server apparatus 100, and generates two-dimensional image 314 of the dental arch as viewed from any point of view based on the accumulated pieces of difference data 119 (S310). Specifically, client apparatus 300 produces combined data B obtained by joining difference data A and difference data B to the display mesh data as shown in Fig. 5, and generates two-dimensional image 314 of the dental arch scanned from the point of view input by the operator with mouse 652. Client apparatus 300 causes display device 500 to display the generated two-dimensional image 314 (S311).

Then, upon acceptance of the operation to end scanning by the operator, scanner 200 ends acquisition of scan data 112 (three-dimensional data) of the object inside the oral cavity (S202). Scanner 200 transmits, to server apparatus 100, new scan data 112 that has been acquired from the previous transmission of scan data 112 to server apparatus 100 until the end of scanning. Server apparatus 100 converts scan data 112 newly transmitted from scanner 200 into point cloud data 114 (conversion into point cloud data: S112). Server apparatus 100 joins two pieces of data based on the overlapping portion between combined data 118 and the newly acquired scan data 112 to generate new combined data 118 (S113).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S312). Client apparatus 300 causes display device 500 to display the generated two-dimensional image 314 (S313).

After the end of scanning, server apparatus 100 generates storage mesh data 120 by using all of the pieces of scan data 112 acquired by scanner 200 (S114). When generating storage mesh data 120, the process of converting all pieces of scan data 112 into point cloud data 114 may be performed in combination with a process of removing noise points from point cloud data 114, a process of smoothing point cloud data 114, a process of aligning all pieces of point cloud data 114, and the like. Server apparatus 100 performs a process of minimizing the error in joining the pieces of scan data 112, for example, by applying a pose graph optimization method on all pieces of scan data 112 after the end of scanning. Thereby, server apparatus 100 can acquire three-dimensional data of the dental arch that ensures the accuracy necessary for digitally designing a prosthesis or the like on a computer. Further, server apparatus 100 may entirely align point cloud data 114 of the dental arches in the upper and lower jaws, and transmit the result to client apparatus 300 as bite alignment information.

Server apparatus 100 causes storage 110 to store the generated storage mesh data 120 (S115). When server apparatus 100 generates storage mesh data 120, it transmits the generated storage mesh data 120 to client apparatus 300. Client apparatus 300 accumulates pieces of storage mesh data 120 transmitted from server apparatus 100, and generates two-dimensional image 314 of the dental arch as viewed from any point of view based on the accumulated pieces of storage mesh data 120 (S314). Client apparatus 300 causes display device 500 to display the generated two-dimensional image 314 (S315). Server apparatus 100 may transmit storage mesh data 120 stored in storage 110 to an external device such as a cloud server as required.

### [Changing Process during Scanning]

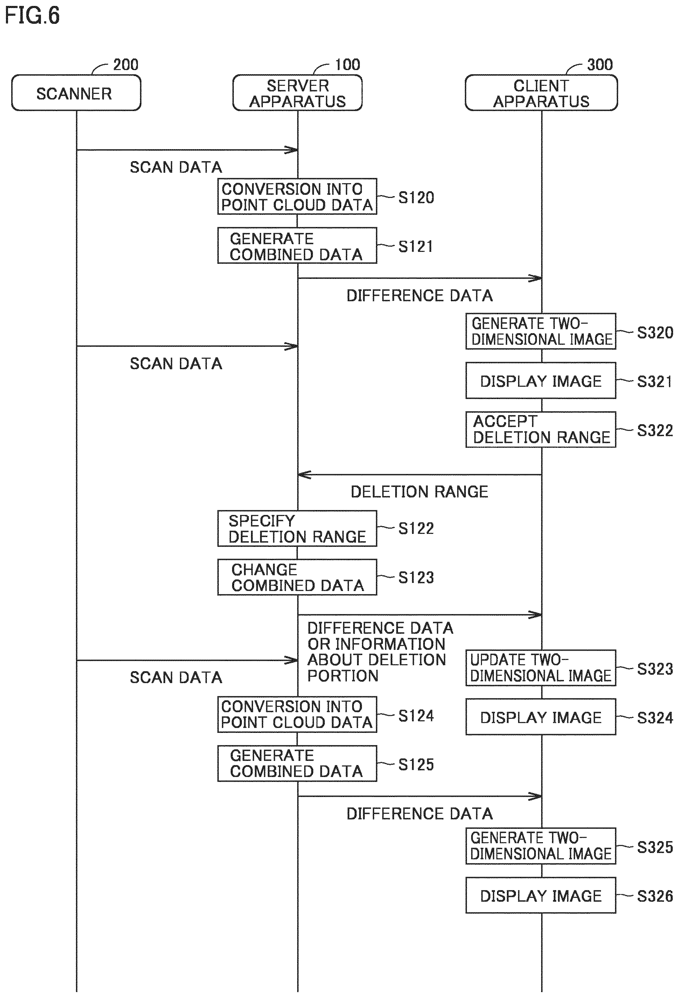

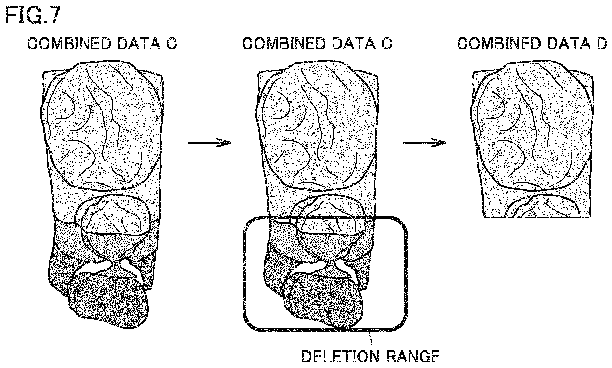

The following describes a changing process performed during scanning in three-dimensional scanner system 1 with reference to a sequence diagram. First, a deletion process as one of the changing process will be hereinafter described. Fig. 6 is a sequence diagram for illustrating a deletion process in three-dimensional scanner system 1 according to the embodiment. Fig. 7 is a schematic diagram for illustrating an example of the deletion process in three-dimensional scanner system 1 according to the embodiment. Herein, "deletion" may include, in addition to completely deleting data, transferring three-dimensional data of an object from a storage area storing the three-dimensional data to another storage area. In other words, "deletion" means deletion or transfer of three-dimensional data of an object from a storage area storing the three-dimensional data.

Server apparatus 100 converts scan data 112 newly transmitted from scanner 200 into point cloud data 114 (conversion into point cloud data: S120). Server apparatus 100 joins two pieces of data based on an overlapping portion between combined data 118 and the newly acquired scan data 112, to generate new combined data 118 (S121). Specifically, server apparatus 100 generates combined data C obtained by joining the scan data to the combined data based on the overlapping portion between the combined data and the scan data as shown in Fig. 7.

When generating combined data 118, server apparatus 100 generates, as difference data 119, a difference between pieces of combined data 118 obtained before and after joining the pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S320). Client apparatus 300 causes display device 500 to display the generated two-dimensional image 314 (S321).

However, when the operator who sees two-dimensional image 314 displayed on display device 500 desires to delete part of combined data 118 for a reason such as conducting scanning again, the operator can designate a deletion range of combined data 118 by using mouse 652. Client apparatus 300 accepts the deletion range of combined data 118 that has been designated by the operator using mouse 652 (S322). Specifically, client apparatus 300 allows the operator to designate the deletion range in combined data C, as shown in Fig. 7. Further, client apparatus 300 transmits the designated deletion range of combined data 118 to server apparatus 100.

Based on the deletion range transmitted from client apparatus 300, server apparatus 100 specifies the range of data to be deleted from combined data 118 (S122). Server apparatus 100 changes the current combined data 118 into combined data 118 from which the specified range of data has been deleted (S123). Specifically, server apparatus 100 changes combined data C into combined data D from which the deletion range has been deleted, as shown in Fig. 7. Further, server apparatus 100 generates, as difference data 119, a difference between pieces of combined data 118 obtained before and after being changed by the deletion process, and transmits difference data 119 to client apparatus 300. Note that server apparatus 100 may transmit the information about the surfaces and the points of the deletion portion (the information about the deletion portion) changed by the deletion process to client apparatus 300 without generating difference data 119 between pieces of combined data 118 obtained before and after being changed by the deletion process. **In** this way, server apparatus 100 transmits only the information about the deletion portion to client apparatus 300, so that the amount of data to be transmitted to client apparatus 300 can be reduced. **In** this case, the information about the deletion portion includes information such as the vertex numbers, the surface numbers and the like of the mesh data.

Client apparatus 300 updates two-dimensional image 314 from which the deletion range has been deleted based on difference data 119 generated by the deletion process (S323). Upon receipt of the information about the deletion portion instead of difference data 119, client apparatus 300 updates two-dimensional image 314 from which the deletion range has been deleted based on the information about the deletion portion. Client apparatus 300 causes display device 500 to display the updated two-dimensional image 314 (S324).

Further, server apparatus 100 converts scan data 112 newly transmitted from scanner 200 into point cloud data 114 (conversion into point cloud data: S124). Server apparatus 100 joins two pieces of data based on the overlapping portion between combined data 118 obtained after the deletion process and the newly acquired scan data 112, to generate new combined data 118 (S125).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S325). Client apparatus 300 causes display device 500 to display the generated two-dimensional image 314 (S326).

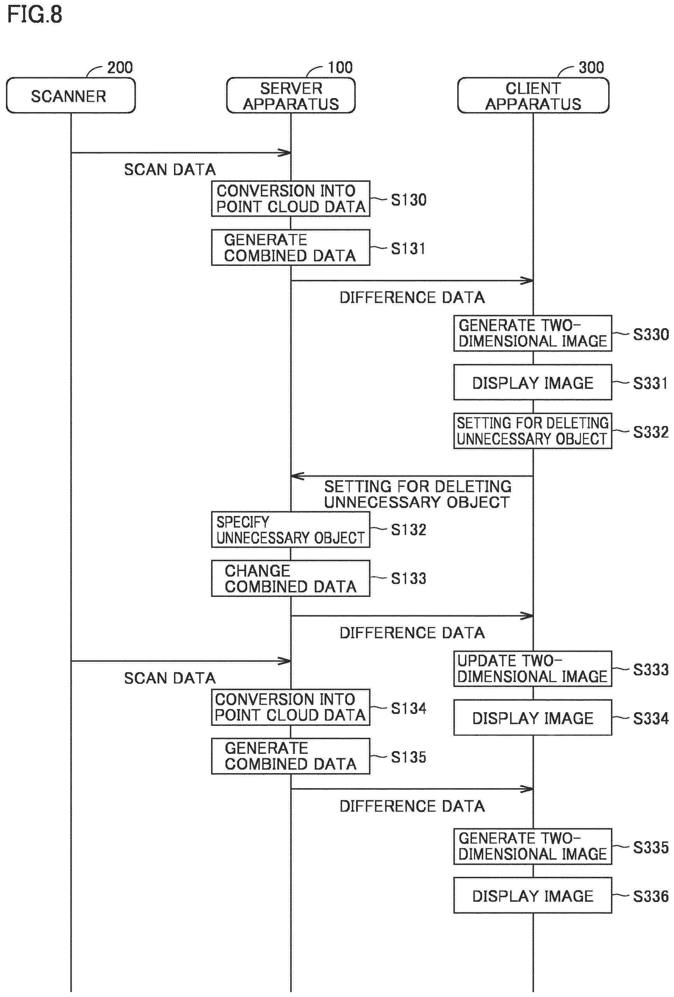

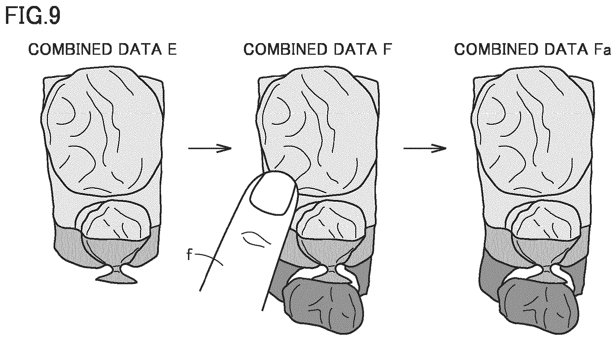

The following describes an unnecessary object deletion process as one of the changing process. Fig. 8 is a sequence diagram for illustrating the unnecessary object deletion process in three-dimensional scanner system 1 according to the embodiment. Fig. 9 is a schematic diagram for illustrating an example of the unnecessary object deletion process in three-dimensional scanner system 1 according to the embodiment.

Server apparatus 100 converts scan data 112 newly transmitted from scanner 200 into point cloud data 114 (conversion into point cloud data: S130). Server apparatus 100 joins two pieces of data based on the overlapping portion between combined data 118 and the newly acquired scan data 112, to generate new combined data 118 (S131). Specifically, as shown in Fig. 9, server apparatus 100 generates combined data E obtained by joining the scan data to the combined data based on the overlapping portion between the combined data and the scan data.

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S330). Client apparatus 300 causes display device 500 to display the generated two-dimensional image 314 (S331).

However, an unnecessary object (for example, a finger, a treatment instrument, or the like) may be captured during scanning of the dental arch and may be shown in two-dimensional image 314 displayed on display device 500. Specifically, as shown in Fig. 9, a finger f as an unnecessary object is shown in combined data F. Thus, when the operator desires to delete the unnecessary object, the operator can select the setting through client apparatus 300 for automatically removing the unnecessary object. Client apparatus 300 accepts the information about the setting for automatically removing the unnecessary object that has been selected by the operator using mouse 652 (S332). The setting for automatically removing the unnecessary object can also be selected before the start of scanning. Further, client apparatus 300 transmits, to server apparatus 100, the information about the setting for removing an unnecessary object.

Based on the information about the setting for removing an unnecessary object that has been transmitted from client apparatus 300, server apparatus 100 specifies the unnecessary object shown in an image from the combined data, on the basis of deletion data 122 of the unnecessary object set in advance (S132). Server apparatus 100 changes the current combined data into combined data 118 from which the data of the specified unnecessary object has been deleted (S133). Specifically, as shown in Fig. 9, server apparatus 100 changes combined data F into combined data Fa from which finger f has been deleted. Further, server apparatus 100 generates, as difference data 119, a difference between pieces of combined data 118 obtained before and after being changed by the unnecessary object deletion process, and then, transmits difference data 119 to client apparatus 300.

Client apparatus 300 updates two-dimensional image 314 based on difference data 119 generated by the unnecessary object deletion process (S333). Client apparatus 300 causes display device 500 to display the updated two-dimensional image 314 (S334).

Further, server apparatus 100 converts scan data 112 newly transmitted from scanner 200 into point cloud data 114 (conversion into point cloud data: S134). Server apparatus 100 joins two pieces of data based on the overlapping portion between combined data 118 obtained after the unnecessary object deletion process and the newly acquired scan data 112, to generate new combined data 118 (S135).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S335). Client apparatus 300 causes display device 500 to display the generated two-dimensional image 314 (S336).

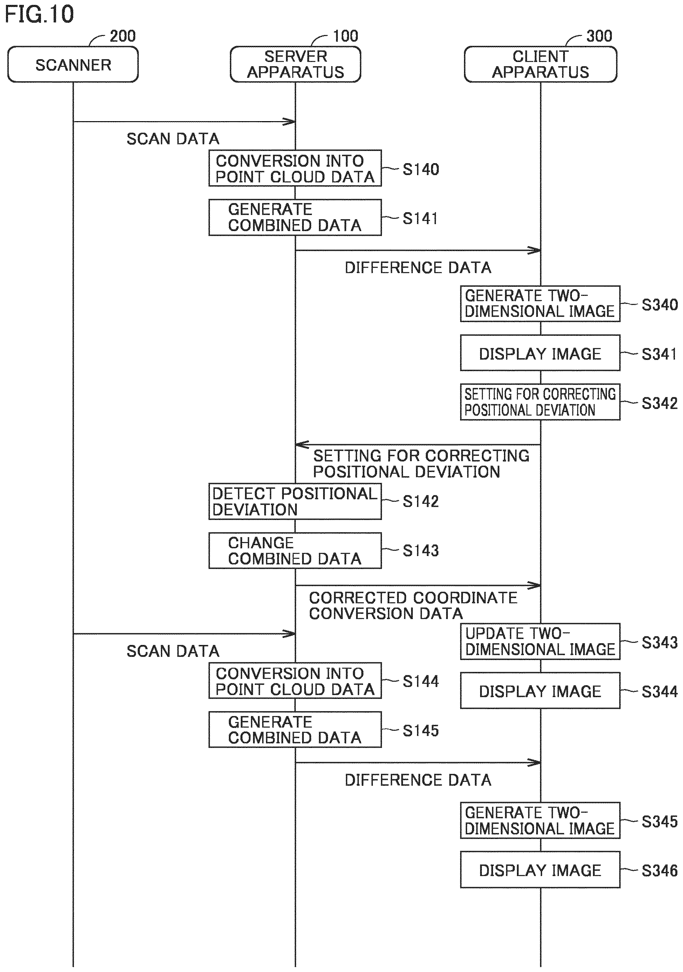

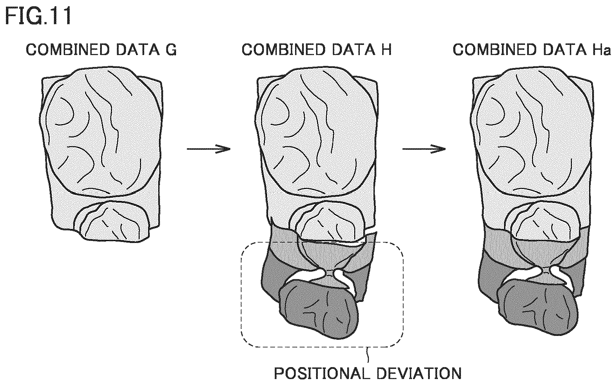

The following describes a positional deviation correction process as one of the changing process. Fig. 10 is a sequence diagram for illustrating the positional deviation correction process in three-dimensional scanner system 1 according to the embodiment. Fig. 11 is a schematic diagram for illustrating an example of the positional deviation correction process in three-dimensional scanner system 1 according to the embodiment.

Server apparatus 100 converts scan data 112 newly transmitted from scanner 200 into point cloud data 114 (conversion into point cloud data: S140). Server apparatus 100 joins two pieces of data together based on the overlapping portion between combined data 118 and the newly acquired scan data 112 to generate new combined data 118 (S141). Specifically, as shown in Fig. 11, server apparatus 100 generates combined data G obtained by joining the scan data to the combined data based on the overlapping portion between the combined data and the scan data.

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S340). Client apparatus 300 causes display device 500 to display the generated two-dimensional image 314 (S341).

However, two-dimensional image 314 displayed on display device 500 includes a portion having a positional deviation of the three-dimensional shape in combined data 118. Specifically, as shown in Fig. 11, combined data H includes a portion having a positional deviation of the three-dimensional shape. Thus, when the operator desires to correct the positional deviation of the three-dimensional shape, the operator can select the setting through client apparatus 300 for automatically correcting the positional deviation of the three-dimensional shape. Client apparatus 300 accepts the information about the setting for automatically correcting the positional deviation of the three-dimensional shape that has been selected by the operator using mouse 652 (S342). The setting for automatically correcting the positional deviation of the three-dimensional shape can also be selected before the start of scanning. Further, client apparatus 300 transmits, to server apparatus 100, the information about the setting for automatically correcting the positional deviation of the three-dimensional shape.

Based on the information about the setting for automatically correcting the positional deviation of the three-dimensional shape that has been transmitted from client apparatus 300, server apparatus 100 detects the position where the positional deviation of the three-dimensional shape occurs in combined data 118 (S142). Note that a known algorithm can be used for an algorithm for correcting the positional deviation of the three-dimensional shape. Server apparatus 100 changes the current combined data 118 into combined data 118 in which the detected position of the positional deviation of the three-dimensional shape has been corrected (S143). Specifically, as shown in Fig. 11, server apparatus 100 changes combined data H into combined data Ha in which the position of the positional deviation of the three-dimensional shape has been corrected. Further, server apparatus 100 generates corrected coordinate conversion data from pieces of combined data 118 obtained before and after being changed by the positional deviation correction process, and transmits the corrected coordinate conversion data to client apparatus 300. Note that server apparatus 100 may generate, as difference data 119, the difference between pieces of combined data 118 obtained before and after being changed and transmit difference data 119 to client apparatus 300, in which case, however, the amount of data to be transmitted is larger than that in the case of transmitting the corrected coordinate conversion data. Herein, the corrected coordinate conversion data is, for example, corresponding pieces of data of the coordinates obtained before and after correction, a conversion function of the coordinates, and the like.

Based on the corrected coordinate conversion data generated by the positional deviation correction process, client apparatus 300 updates the coordinates at each of points of two-dimensional image 314 before correction to the coordinates at each of points of two-dimensional image 314 after correction (S343). Client apparatus 300 causes display device 500 to display the updated two-dimensional image 314 (S344).

Further, server apparatus 100 converts scan data 112 newly transmitted from scanner 200 into point cloud data 114 (conversion into point cloud data: S144). Server apparatus 100 joins two pieces of data based on the overlapping portion between combined data 118 obtained after the positional deviation correction process and the newly acquired scan data 112, to generate new combined data 118 (S145).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S345). Client apparatus 300 causes display device 500 to display the generated two-dimensional image 314 (S346).

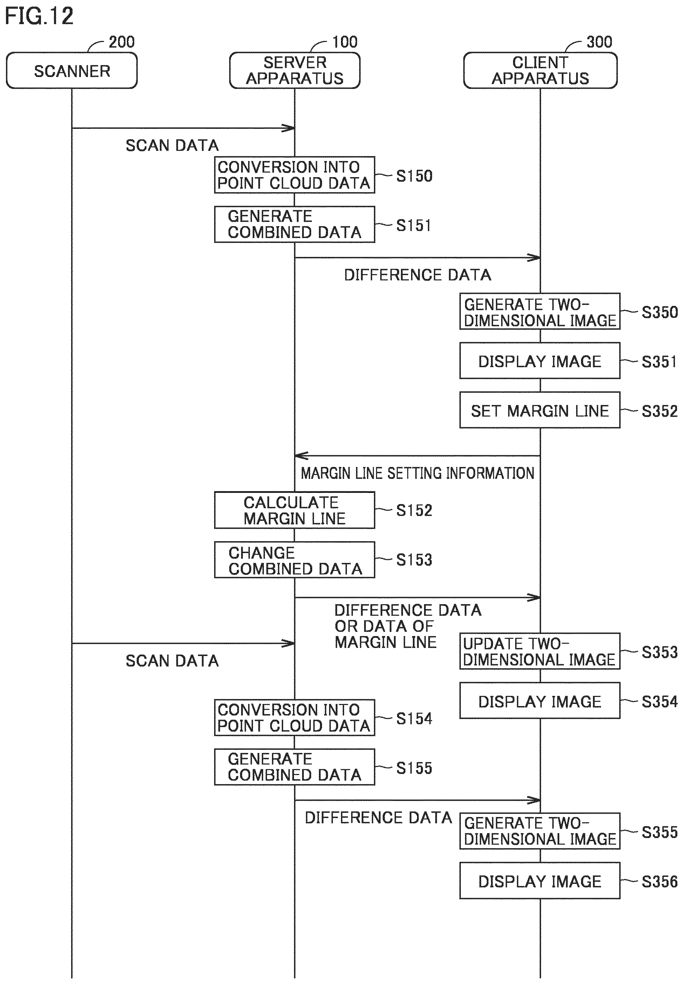

The following describes a margin line process as one of the changing process. Fig. 12 is a sequence diagram for illustrating a margin line process in three-dimensional scanner system 1 according to the embodiment. Herein, the margin line process is a process of clarifying edges of a restoration and a prosthesis in three-dimensional data of a dental arch.

Server apparatus 100 converts scan data 112 newly transmitted from scanner 200 into point cloud data 114 (conversion into point cloud data: S150). Server apparatus 100 joins two pieces of data based on the overlapping portion between combined data 118 and the newly acquired scan data 112, to generate new combined data 118 (S151).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S350). Client apparatus 300 causes display device 500 to display the generated two-dimensional image 314 (S351).

However, in combined data 118 of the dental arch generated from the acquired scan data 112, the edges of the restoration or the prosthesis may be ambiguous. Thus, the operator can determine the margin line in combined data 118 of the dental arch while seeing two-dimensional image 314 displayed on display device 500. Client apparatus 300 accepts the information about the operator's selection, through mouse 652, of one or more points in combined data 118 that are considered to be edges of the restoration or the prosthesis (set a margin line: S352). Further, client apparatus 300 transmits, to server apparatus 100, margin line setting information including the information about one or more points selected by the operator.

Server apparatus 100 calculates a margin line in combined data 118 based on the margin line setting information transmitted from client apparatus 300 (S152). Note that a known algorithm can be used for an algorithm for calculating the margin line. Server apparatus 100 changes the current combined data 118 into combined data 118 including the calculated margin line (S153). Further, server apparatus 100 generates, as difference data 119, a difference between pieces of combined data 118 obtained before and after being changed by the margin line process, and transmits difference data 119 to client apparatus 300. In step S153, server apparatus 100 may transmit only the data of the margin line to client apparatus 300 without changing into combined data 118 including the calculated margin line.

Client apparatus 300 updates two-dimensional image 314 based on difference data 119 generated by the margin line process (S353). Client apparatus 300 causes display device 500 to display the updated two-dimensional image 314 (S354).

Further, server apparatus 100 converts scan data 112 newly transmitted from scanner 200 into point cloud data 114 (conversion into point cloud data: S154). Server apparatus 100 joins two pieces of data based on the overlapping portion between combined data 118 obtained after the margin line process and the newly acquired scan data 112, to generate new combined data 118 (S155).

When generating combined data 118, server apparatus 100 generates, as difference data 119, a difference between pieces of combined data 118 obtained before and after joining pieces of scan data 112 together, and transmits difference data 119 to client apparatus 300. Client apparatus 300 accumulates pieces of difference data 119 transmitted from server apparatus 100, and generates two-dimensional image 314 of the dental arch as viewed from any point of view based on the accumulated pieces of difference data 119 (S355). Client apparatus 300 causes display device 500 to display the generated two-dimensional image 314 (S356). In the description of the margin line process shown in Fig. 12, the margin line is calculated using a known algorithm before production of the storage mesh data shown in Fig. 4, but the present disclosure is not limited thereto, and the margin line may be calculated after production of the storage mesh data.

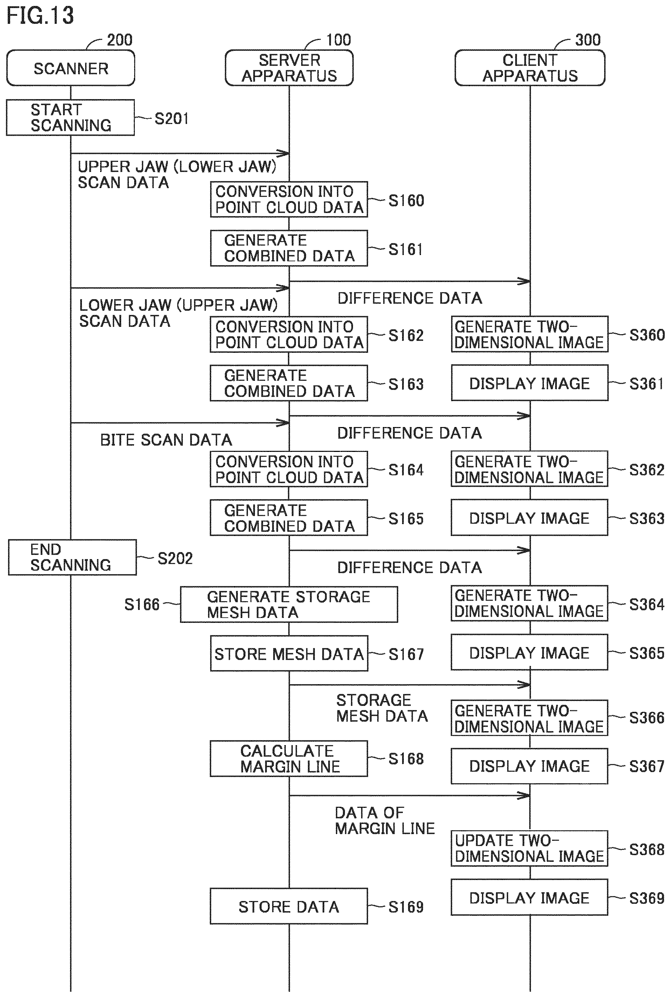

More specifically, in three-dimensional scanner system 1, when a process of scanning the patient's dental arch is performed using scanner 200, the scanning process is performed, for example, in a workflow of: upper jaw (or lower jaw) scanning, lower jaw (or upper jaw) scanning, bite scanning, a post process (production of storage mesh data), an analysis, a margin line process, and storage. Fig. 13 is a sequence diagram for illustrating an example of a dental arch scanning process in three-dimensional scanner system 1 according to the embodiment. First, upon acceptance of an operation to start scanning by an operator, scanner 200 starts acquisition of scan data 112 (three-dimensional data) of an object inside an oral cavity (S201).

Scanner 200 scans the dental arch of the upper jaw (or the lower jaw) inside the oral cavity, sequentially acquires pieces of scan data 112 of the dental arch of the upper jaw (or the lower jaw), and sequentially transmits the acquired pieces of scan data 112 to server apparatus 100. Server apparatus 100 converts the pieces of scan data 112 sequentially transmitted from scanner 200 into point cloud data 114 (conversion into point cloud data: S160). Server apparatus 100 first generates display mesh data 116 corresponding to point cloud data 114, and subsequently generates combined data 118 obtained by joining two pieces of data based on the portion overlapping with the newly acquired scan data 112 (S161). Server apparatus 100 transmits display mesh data 116 to client apparatus 300 and subsequently transmits, to client apparatus 300, difference data 119 between pieces of combined data 118 obtained before and after joining pieces of scan data 112 together.

Based on the received display mesh data 116 or difference data 119, client apparatus 300 generates two-dimensional image 314 of the scanned dental arch of the upper jaw (or the lower jaw) as viewed from any point of view (S360). Client apparatus 300 causes display device 500 to display the generated two-dimensional image 314 (S361).

Then, scanner 200 scans the dental arch of the lower jaw (or the upper jaw) inside the oral cavity, sequentially acquires pieces of scan data 112 of the dental arch of the lower jaw (or the upper jaw), and sequentially transmits the acquired pieces of scan data 112 to server apparatus 100. Server apparatus 100 converts the pieces of scan data 112 sequentially transmitted from scanner 200 into point cloud data 114 (conversion into point cloud data: S162). Server apparatus 100 first generates display mesh data 116 corresponding to point cloud data 114 and subsequently generates combined data 118 obtained by joining two pieces of data based on the portion overlapping with the newly acquired scan data 112 (S163). Server apparatus 100 transmits display mesh data 116 to client apparatus 300 and subsequently transmits, to client apparatus 300, difference data 119 between pieces of combined data 118 obtained before and after joining pieces of scan data 112 together.

Based on the received display mesh data 116 or difference data 119, client apparatus 300 generates two-dimensional image 314 of the scanned dental arch of the lower jaw (or the upper jaw) as viewed from any point of view (S362). Client apparatus 300 causes display device 500 to display the generated two-dimensional image 314 (S363).

Then, scanner 200 scans the bite between the dental arches inside the oral cavity to sequentially acquire pieces of scan data 112 of the bite between the dental arches, and sequentially transmits the acquired pieces of scan data 112 to server apparatus 100. Server apparatus 100 converts the pieces of scan data 112 sequentially transmitted from scanner 200 into point cloud data 114 (conversion into point cloud data: S164). Server apparatus 100 first generates display mesh data 116 corresponding to point cloud data 114, and subsequently generates combined data 118 obtained by joining two pieces of data based on the portion overlapping with the newly acquired scan data 112 (S165). Server apparatus 100 transmits display mesh data 116 to client apparatus 300 and subsequently transmits, to client apparatus 300, difference data 119 between pieces of combined data 118 obtained before and after joining pieces of scan data 112 together.

Based on the received display mesh data 116 or difference data 119, client apparatus 300 generates two-dimensional image 314 of the bite between the scanned dental arches as viewed from any point of view (S364). Client apparatus 300 causes display device 500 to display the generated two-dimensional image 314 (S365).

Then, upon acceptance of an operation to end scanning by the operator, scanner 200 ends acquisition of scan data 112 (three-dimensional data) of the object inside the oral cavity (S202). After the end of scanning, server apparatus 100 generates storage mesh data 120 by using all of the pieces of scan data 112 acquired by scanner 200 (S166). Server apparatus 100 causes storage 110 to store the generated storage mesh data 120 (S167).

When server apparatus 100 generates storage mesh data 120, it transmits storage mesh data 120 to client apparatus 300. Client apparatus 300 accumulates pieces of storage mesh data 120 transmitted from server apparatus 100, and generates two-dimensional image 314 of the dental arch as viewed from any point of view based on the accumulated pieces of storage mesh data 120 (S366). Client apparatus 300 causes display device 500 to display the generated two-dimensional image 314 (S367).

Although not shown in Fig. 13, server apparatus 100 may then perform an analysis process desired by the user. Further, server apparatus 100 calculates a margin line in combined data 118 based on the margin line setting information transmitted from client apparatus 300 (S168). Server apparatus 100 transmits the data of the calculated margin line to client apparatus 300.

Based on the data of the margin line calculated by the margin line process, client apparatus 300 updates two-dimensional image 314 of the dental arch including the margin line (S368). Client apparatus 300 causes display device 500 to display the updated two-dimensional image 314 (S369).

At the end of the dental arch scanning process, server apparatus 100 causes storage 110 to store the analyzed data, the data of the calculated margin line, and the like in addition to storage mesh data 120 (S169).

When a process such as trimming is performed in three-dimensional scanner system 1, each trimming is performed after each scanning, for example, in a sequence of: upper jaw (or lower jaw) scanning; trimming; lower jaw (or upper jaw) scanning; and trimming, but trimming is not performed after a post process (production of storage mesh data). Without being limited thereto, however, three-dimensional scanner system 1 may perform trimming after the end of all scanning processes and before the post process (production of storage mesh data) or may perform trimming after the post process (production of storage mesh data).

In the description of three-dimensional scanner system 1, the margin line process is performed after the post process (production of storage mesh data) but may be performed before the post process (production of storage mesh data). In three-dimensional scanner system 1, various kinds of processes should only be performed between server apparatus 100 and client apparatus 300 irrespective of the production of the storage mesh data.

### [Modifications]

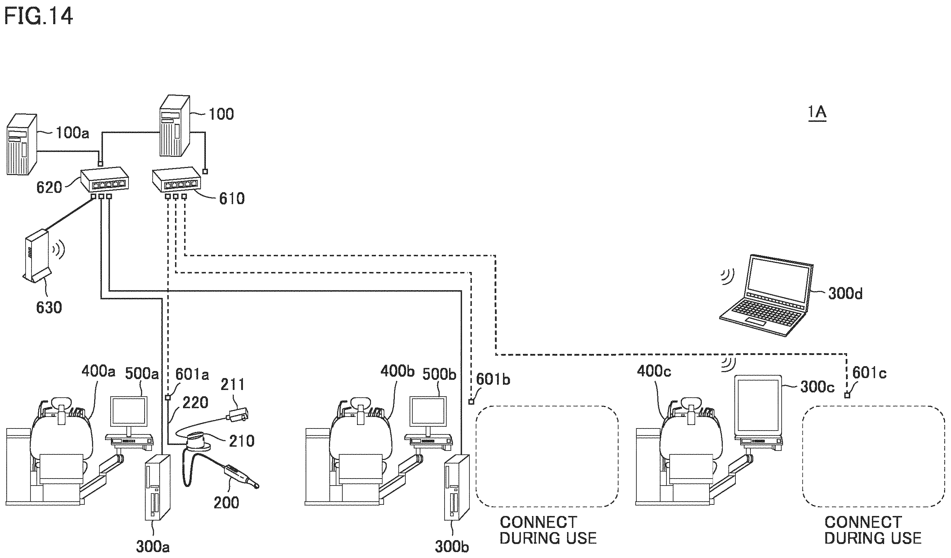

The configuration of the three-dimensional scanner system is not limited to the configuration shown in Fig. 1, but the three-dimensional scanner system can have various configurations. Hereinafter, a modification of the configuration of the three-dimensional scanner system will be described with reference to the accompanying drawings. Fig. 14 is a schematic diagram showing a configuration of a three-dimensional scanner system 1A according to the modification. In three-dimensional scanner system 1A, three chair units 400a to 400c are provided in a clinic. When one scanner 200 is used, LAN cable 220 is connected to insertion ports 601a to 601c provided in respective chair units 400a to 400c.

Insertion ports 601a to 601c are network-connected to server apparatus 100 via a network hub 610. When network hub 610 has the PoE function, electric power can be supplied to scanner 200 through LAN cable 220. However, when network hub 610 does not have the PoE function, electric power is supplied to scanner 200 and cradle 210 through the connection of an AC adapter 211 to cradle 210 as shown in Fig. 14.

In three-dimensional scanner system 1A, client apparatuses 300a and 300b provided in respective chair units 400a and 400b are network-connected to server apparatus 100 via a network hub 620. A management server apparatus 100a and an access device 630 for wireless communication are further connected to network hub 620. In a clinic, before three-dimensional scanner system 1A is introduced, management server apparatus 100a and client apparatuses 300a and 300b have already been introduced, in which management server apparatus 100a can manage patient information, image data, examination data, electronic medical records, and the like, and cause display devices 500a and 500b provided in respective chair units 400a and 400b to display the patient information and the like via respective client apparatuses 300a and 300b as necessary.

In three-dimensional scanner system 1A, the data of the three-dimensional shape processed by server apparatus 100 can be displayed on display devices 500a and 500b by using respective client apparatuses 300a and 300b that are already installed. Further, by introducing a Web application program running on a Web browser into display devices 500a and 500b, the already installed client apparatuses 300a and 300b can be directly used without installing a dedicated application program, so that the cost for introducing three-dimensional scanner system 1A can be reduced.

Further, it is not necessary to provide a stationary client apparatus like chair units 400a and 400b, but a tablet terminal 300c or a notebook PC 300d may be provided in chair unit 400c as shown in Fig. 14. Tablet terminal 300c and notebook PC 300d are not connected to network hub 620 by wire, but are connected to access device 630 through wireless communication.

In three-dimensional scanner system 1A, for example, a workstation of a general-purpose image diagnosis apparatus is connected to cooperate with network hub 620, to allow management of the three-dimensional data of the dental arch in association with an X-ray image, a computed tomography (CT) image, an optical coherence tomography (OCT) image, and the like. Further, in three-dimensional scanner system 1A, for example, if management server apparatus 100a functions as a receipt computer or an electronic medical record system, the three-dimensional data of the dental arch can be managed in association with the patient information, the examination data, the medical fee receipts, the electronic medical records, and the like. Further, in three-dimensional scanner system 1A, data obtained from other devices such as an intra-oral camera and a face scanner may also be managed in association therewith.

In three-dimensional scanner system 1A, server apparatus 100 and scanner 200 are connected by wire, but may be connected through wireless communication. In three-dimensional scanner system 1A, one scanner 200 is installed in a clinic, so that server apparatus 100 and scanner 200 are connected on a one-to-one basis. In three-dimensional scanner system 1A, however, a plurality of scanners 200 may be introduced in a clinic, and server apparatus 100 and a plurality of scanners 200 may be connected on a one-to-many basis.

Three-dimensional scanner system 1A may be able to cause display device 500 to display a cross-sectional view of the three-dimensional shape of the scanned dental arch. Further, three-dimensional scanner system 1A may be able to measure the clearance between the upper and lower jaws in an occlusal state of the scanned dental arches. Three-dimensional scanner system 1A may be able to cause display device 500 to display an undercut portion of the scanned dental arch as viewed in a specific direction. Three-dimensional scanner system 1A may be able to measure any distance and angle of the three-dimensional shape of the scanned dental arch.

Three-dimensional scanner system 1A may be configured to cooperate with dental engineering instruction generation software. Further, three-dimensional scanner system 1A may be configured to cooperate with software or a service by which three-dimensional data, data such as a dental engineering instruction manual, messages, and the like are transmitted to and received from a dental laboratory.

Display device 500 is a display provided in each of chair units 400a and 400b but may be a display provided separately from each of chair units 400a and 400b. Three-dimensional scanner system 1A may employ a display of a notebook PC, a display of a tablet terminal, or a head-mounted display instead of display device 500.

In each of three-dimensional scanner systems 1 and 1A, data such as scan data 112, combined data 118, and storage mesh data 120 may be stored in a large-capacity storage installed in a clinic or a storage on a cloud.

It should be understood that the disclosure herein is illustrative and non-restrictive in every respect. The scope of the present disclosure is defined by the claimed subject-matter, rather than the description above. Note that the configurations illustrated in the embodiments and the configurations illustrated in the modifications can be combined as appropriate.

## Claims

1. An information processing system configured to process three-dimensional data, the information processing system comprising:
a scanner configured to scan an object to sequentially acquire pieces of three-dimensional data of the object;
a server apparatus configured to, when joining pieces of three-dimensional data based on an overlapping portion of the pieces of three-dimensional data sequentially transmitted from the scanner and generating combined data indicating a three-dimensional shape of the object, generate, as difference data, a difference between pieces of the combined data obtained before and after joining the pieces of three-dimensional data together; and
a client apparatus configured to accumulate pieces of the difference data sequentially transmitted from the server apparatus, and generate a two-dimensional image of the object as viewed from any point of view based on the accumulated pieces of the difference data.

2. The information processing system according to claim 1, wherein
the scanner is configured to acquire three-dimensional data of the object inside an oral cavity, and
the object is a dental arch inside the oral cavity or a model of the dental arch.

3. The information processing system according to claim 1 or 2, wherein the three-dimensional data is point cloud data composed of points each indicating a three-dimensional coordinate position.

4. The information processing system according to any one of claims 1 to 3, wherein the server apparatus is configured to process the combined data into mesh data.

5. The information processing system according to any one of claims 1 to 4, wherein
the client apparatus includes an input unit configured to accept a user operation, and
the server apparatus is configured to perform a changing process on the combined data based on the user operation accepted by the input unit.

6. The information processing system according to claim 5, wherein
the server apparatus is configured to generate, as the difference data, a difference between pieces of the combined data obtained before and after being changed by the changing process, and
the client apparatus is configured to update the two-dimensional image based on the difference data generated by the changing process.

7. The information processing system according to claim 5 or 6, wherein, in the changing process, when the input unit accepts information that a user designates a range to be deleted in the combined data, the server apparatus changes the combined data into combined data from which data of the range to be deleted has been deleted.

8. The information processing system according to any one of claims 5 to 7, wherein, in the changing process, when the input unit accepts information about a setting for automatically removing an unnecessary object, the server apparatus specifies deletion data of the unnecessary object set in advance from the combined data, and changes the combined data into combined data from which the specified deletion data has been deleted.

9. The information processing system according to any one of claims 5 to 8, wherein, in the changing process, when the input unit accepts information about a setting for automatically correcting a positional deviation of a three-dimensional shape in the combined data, the server apparatus detects the positional deviation and changes the combined data into combined data in which the detected positional deviation has been corrected.

10. The information processing system according to any one of claims 5 to 9, wherein, in the changing process, when the input unit accepts information that a user selects one or more points in the combined data, the server apparatus calculates a margin line including selected points, and changes the combined data into combined data including the margin line or generates data of the margin line without changing the combined data.

11. The information processing system according to any one of claims 1 to 10, wherein
the server apparatus has a function as a Web server, and is configured to, when the Web server generates the combined data, generate the difference data between before and after joining the pieces of three-dimensional data together, and
the client apparatus is configured to generate the two-dimensional image based on the difference data by a process of a Web application.

12. The information processing system according to any one of claims 1 to 10, wherein the server apparatus is configured to, during scanning of the object by the scanner, join some of the pieces of three-dimensional data sequentially transmitted from the scanner to generate the combined data, and generate the difference data between before and after joining the pieces of three-dimensional data together.

13. The information processing system according to claim 12, wherein the server apparatus is configured to, after scanning of the object by the scanner ends, join all of the pieces of three-dimensional data sequentially transmitted from the scanner to update the combined data.

14. A server apparatus configured to transmit and receive data to and from a client apparatus included in an information processing system configured to process three-dimensional data, the server apparatus comprising:
a reception unit configured to receive pieces of three-dimensional data sequentially transmitted from a scanner configured to scan an object to sequentially acquire pieces of three-dimensional data of the object;
a computing unit configured to, when joining pieces of three-dimensional data together based on an overlapping portion of the pieces of three-dimensional data sequentially received by the reception unit and generating combined data indicating a three-dimensional shape of the object, generate, as difference data, a difference between pieces of the combined data obtained before and after joining the pieces of three-dimensional data together; and
a transmission unit configured to accumulate pieces of the difference data generated by the computing unit, and transmit the accumulated pieces of the difference data to a client apparatus configured to generate a two-dimensional image of the object as viewed from any point of view based on the accumulated pieces of the difference data.

15. A client apparatus configured to transmit and receive data to and from a server apparatus included in an information processing system configured to process three-dimensional data, the client apparatus comprising:
a reception unit configured to, when joining pieces of three-dimensional data together based on an overlapping portion of pieces of three-dimensional data sequentially transmitted from a scanner configured to scan an object to sequentially acquire pieces of three-dimensional data of the object, and generating combined data indicating a three-dimensional shape of the object, receive difference data transmitted from the server apparatus configured to generate, as the difference data, a difference between pieces of the combined data obtained before and after joining the pieces of three-dimensional data together;
a computing unit configured to accumulate pieces of the difference data received by the reception unit and generate a two-dimensional image of the object as viewed from any point of view based on the accumulated pieces of the difference data; and
a display unit configured to display the two-dimensional image.
